Europäisches Patentamt

European Patent Office

Office européen des brevets

(19)

(11) Publication number: **0 265 690**
**A2**

# (12) EUROPEAN PATENT APPLICATION

(21) Application number: 87114198.2

(51) Int. Cl.4: **C12Q 1/04**

(22) Date of filing: 29.09.87

The title of the invention has been amended (Guidelines for Examination in the EPO, A-III, 7.3).

(30) Priority: 29.09.86 CS 6957/86

(43) Date of publication of application:
**04.05.88 Bulletin 88/18**

(84) Designated Contracting States:
**CH DE FR GB LI SE**

(71) Applicant: **Ceskoslovenska akademie ved**
**Narodni trida 3**
**Praha 1(CS)**

(72) Inventor: **Cermák, Pavel, MU Dr.**
**Stichova 600/29**
**Praha 4(CS)**
Inventor: **Monhart CSh, Václav, MU Dr.**
**U. baterie 206/51**
**Praha 6(CS)**
Inventor: **Horák, Jiri, MU Dr., CSc**
**Podolska 1485**
**147 00 Praha 4(CS)**
Inventor: **Tlustaková, Marie CSc**
**Kozoninská 637**
**Praha 8(CS)**
Inventor: **Paroubek, Miroslav**
**Famfulikova 12**
**Praha 8(CS)**

(74) Representative: **Patentanwälte Beetz sen. -**
**Beetz jun. Timpe - Siegfried -**
**Schmitt-Fumian**
**Steinsdorfstrasse 10**
**D-8000 München 22(DE)**

(54) **Method and device for the determination of the presence of microorganisms in body liquors.**

(57) The invention pertains to a method for the determination of the presence of microorganisms in body liquors and to a diagnosis column for performing this method.

Body liquor is allowed to flow through the column packed with a sorbent, and trapped microorganisms are then cultivated in a culture medium which is charged directly into the column. After activation, the culture medium is sucked off from the column, and the presence of microorganisms is determined by known methods in the column.

The diagnosis column is made from a biocompatible material, has a smooth inner surface advantageously provided with longitudinal ribs (6), its jacket consisting of a cylindrical body (1) and conical terminals (5), and is packed with a sorbent (4) placed between partitions (2, 3) from a porous material, which advantageously have various porosity, whereas the volume ratio of the cylindrical body (1) to that of the conical terminals (5) is in the range of 1:0,3 to 1:5, and the total inner volume is 30 to 300 ml.

The partition (2) at the outlet terminal is advantageously formed from several layers having different porosities.

The diagnosis columns may be used in medicine for trapping and diagnostics of microbial agents responsible for septic states.

# Method for the determination of the presence of microorganisms in body liquors and diagnosis column therefor

The invention pertains to a method for the determination of the presence of microorganisms in body liquors and to a diagnosis column for performing this method.

Numerous serious pyrexiae are caused by microbial infections where the aetiological agent occurs in the circulating blood of the patient in various amounts and for various periods of time. The usual detection method by a single or also repeated sampling into a vessel with culture medium does not render satisfactory results.

An efficient method for the detection of an infective microbial agent causing pyrexia in blood is the diagnostic perfusion. Blood of the examinated person flows through a column packed with sorbent particles for a predetermined period of time. The total blood volume of an adult patient passes through the column in about 60 min at a flow rate of 100•ml min⁻¹.

For this purpose, only one type of diagnosis column has heretofore been used (Keller, F., Feldman, K., Abshagen, U., et al., Klin. Wochenschr. 59 Nr. 9 (1981) 425-429; Matthali, D., Kramer, P., Grieben, K., et al., Contr. Nephrol. 32 (1982) 175-180). This column is packed with surface-modified activated charcoal. Its body is formed by a short and broad cylinder with both ends provided with external threads. Very flat terminals are screwed on the threads and accomodate and seal mouldings acting as screens. For the purpose of activation, this type of column has to be opened by unscrewing the terminal and removing the screen. Then the grains of surface-modified charcoal can be transferred into a culture medium.

It is an object of the present invention to provide a method and a diagnosis column for the determination of the presence of microorganisms in body liquors which avoid the disadvantages connected with dismantling the column and transferring the sorbent into a culture medium outside the column.

The avobe object is achieved according to claims 1 and 2.

The method for the determination of the presence of microorganisms causing septic states in body liquors according to the invention avoids the avobe-mentioned disadvantage. Body liquor is first allowed to flow through a column packed with a sorbent which traps microorganisms. First, the residual body liquor is displaced by physiological saline, whereupon the column is filled with a culture medium in such a way that the all sorbent is immersed, and the cultivation is carried out directly in the column. On completion of the cultivation, the culture medium is discharged from the column, preferably by suction, and the presence of microorganisms is determined by methods known as such.

The diagnosis column for performing the method according to the invention is made of a biocompatible material, has a smooth inner surface, advantageously with longitudinal ribs, is packed with a sorbent placed between partitions made of a porous material, and is characterized by a jacket consisting of a cylindrical body and conical terminals with a mutual volume ratio of 1:0,3 to 1:5, whereby the supporting partitions have different porosity.

According to a preferred embodiment, the diagnosis column according to the invention has a total inner volume of 30 to 300 ml. The volume of the column cylindrical body is about 10 to 100 ml, and the total volume of both terminals is about 20 to 200 ml. The partitions separating the sorbent may have different porosity. The partition near the outlet terminal is advantageously formed of several layers having different porosity, preferably 2000 to 80, 150 to 50, and 80 to 20 μm. The inner surface of column body may advantageously be provided with ribs.

The diagnosis column according to the invention is packed with a sorbent advantageously coated with a protective layer of a bicompatible polymer of methacrylate or acrylate type preferably in an amount of 0,01 to 20 mass-%.

The diagnosis column according to the invention is schematically shown in the drawings, wherein Fig. 1 shows a longitudinal cross-section of the column, and Figs. 2 and 3 show a horizontal cross-section through the column provided with ribs.

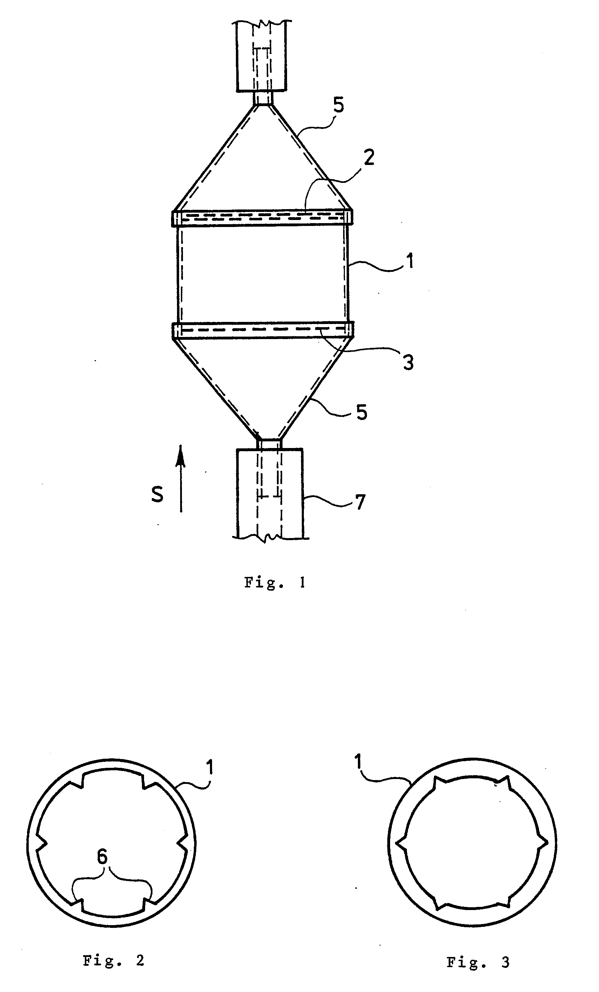

The column according to Fig. 1 consists of a column body 1 which is connected, via porous partitions 2 and 3 separating the sorbent 4, with conical terminals 5 with attached inlet and outlet tubes 7. The arrow S in Fig. 1 indicates the direction of blood flow. An arbitrary number of ribs 6 may be provided.

Figs. 2 and 3 schematically show horizontal cross-sections with marked ribs. An arbitrary number of ribs 6 may be provided.

The column material has to be strong, biocompatible and non-thrombogenic, and has to withstand sterilization. Polypropylene, ethylene - propylene copolymers, polycarbonates, poly(tetrafluoroethylene) and similar materials can be used. These polymers may be processed by injection moulding or extrusion blowing. The shape of the column represents a suitable compromise between the demands on blood through-flow and technical and economical production aspects. The column must have a smooth inner surface advantageously provided with longitudinal ribs 6 which assist in directing the blood flow and reducing the motion of sorbent grains 4, and increase the strength of the jacket. The column body 1 and the conical terminals 5 are designed in such a way that the transitions between them are completely smooth. The connection of the body 1 to the terminal 5 is realized by adhesion, adhesion gluing or by heat, ultrafrequency or ultrasound welding.

The spaces of terminals 5 and column body 1 are separated with screens 2, 3 which act as support of the sorbent 4 inside the column and at the same time prevent from penetration of the sorbent 4 into the terminal 5 or as far as into the blood circulation of the patient. The screens 2, 3 may be made of stainless steel, synthetic fabric, or plastic mouldings. Their material has to meet the same conditions as the material of the column jacket. A polypropylene or polyester fabric or porous polyurethane may be used. Both screens 2, 3 may have the same or different density. It is advantageous to use a denser screen 2 in the outlet terminal where it acts as a protection against the penetration of thrombs, which may form in the column, into the blood circulation of the patient. However, such columns can be used only in one direction, and the blood-flow direction has to be indicated on the jacket. The mesh size of the screen 2 may range from 40 μm to an upper limit which is determined by the grain size of the sorbent 4. The screens 2, 3 may be of multiple type and consist of several layers of different porosity.

The sorbent 4 is packed into the column by means of an isotonic apyrogenic solution of sodium chloride before the outlet terminal with the screen 2 is attached. The sorbent 4 may active charcoal preferably made from coconut shells or a synthetic resin. The optimal particle size is 0,3 to 0,7 mm.

The packed column is then closed with the upper terminal, provided with tubes 7 at both ends and sterilized for 2 h at 120 °C in an autoclave.

Application of the column in the microbiological laboratory:

The diagnosis column contains microbial cells trapped from patient's blood on the surface of the sorbent 4. Their detection and further determination require the cultivation of the sorbent in a culture medium suitable for the growth of the respective microbes. The diagnosis column according to the invention is designed such that the cultivation can be carried out directly in the column in a liquid culture medium. Opening of the column and handling of the sorbent are not necessary, and the possible contamination of the column content and of laboratory personal are prevented.

The tubes 7 at both ends of the column may serve for handling of the liquid content of the column (discharging of the liquid content, charging with a culture medium, inoculation of the grown microbial culture) because they can easily be punctured with a hypodermic needle, and the fluid content may be handled by means of a syringe. An air filter trapping the contingent air contamination can be provided at the end of the tube 7 during discharging the fluid content of the column.

All types of liquid culture media either commercial or prepared in laboratory may be used as the cultivation charge. The volume of the culture medium must be sufficient to immerse or cover all particles of the sorbent 4.

Microorganisms are trapped above all in a biological coating which is formed on the surface of particles present in blood. The system of blood coagulation plays an important role in the formation of this coating. A fibrin network is formed on the surface of the particles which traps blood platelets, a small number of red and white blood cells, and other corpuscles including microorganisms. Besides a simple adherence of microorganisms to the surface of the particles, above all a mechanical trapping of microorganisms in the fibrin network and various specific and non-specific receptors and ligands contribute to this process.

The sorbent particles 4 can be made from an inorganic or organic substance, from their combinations, and from laminated materials, and may have various shape, size and internal structure. The use of a biocompatible material is suitable as it prevents from the formation and growth of thrombs on the particles.

The diagnostic column according to the invention has many advantages in comparison with similar columns heretofore used. The firm connection of the column body 1 with the terminals 5 warrants the necessary sterility of column much better than the commonly used screw closure with uncertain tightness. This substantially extends the expiration time, i.e. the period of column applicability, which is advantageous from the point of view of production as well as of clinical practice. The column according to the invention is designed such that it allows cultivation of trapped bacteria without opening and handling of the sorbent. It is suited for working in a closed and strictly sterile system which excludes the possibility of contamination from outside and deterioration of the test results. At the same time, the risk of infection of medical and laboratory attendances is reduced to a minimum. Further, it enables optimal conditions for trapping and cultivation of bacteria from blood and thus substantially improves the diagnosis of septic states. The invention is further described by way of examples.

## Example 1

A diagnosis column having a volume of 50 ml made from polypropylene (screens with a mesh size of 290 $\mu$m) and packed with active charcoal from coconut shells (Chemviron SC XII) with an untreated surface was used in an experiment with an animal infected with rod-like Escherichia coli bacteria.

The haemoperfusion was carried out for 1 h at a rate of 20 ml/min. On conclusion of the haemoperfusion, the tube was punctured at the end of the column, and the liquid content was removed by suction with a hypodermic syringe and needle. The column was then charged in the same way with a culture medium (liver stock) in such amount that the sorbent was completely immersed. The column charged with the culture medium was kept for 24 h at 37 °C. After completion of the activation, a sample of the culture medium was withdrawn in the same manner as the column was earlier discharged. The propagated bacteria were identified microscopically and by further cultivation on solid cultivation substrates.

## Example 2

A diagnosis column having a volume of 30 ml made from polypropylene (screens with a mesh size of 300 $\mu$m) and packed with active charcoal having the surface modified with 3 mass-% of poly-(2-hydroxyethyl methacrylate) was used in an experiment with an animal infected with rod-like Escherichia coli bacteria. The haemoperfusion was carried out for 1 h at a rate of 20 ml/min. After 24 h of cultivation, the bacteria were then isolated from a culture medium in the column.

## Example 3

A diagnosis column having a volume of 70 ml made from polypropylene (screens with a mesh size of 300 $\mu$m) and packed with active charcoal from coconut shells (Chemviron SC II) having the surface modified with 3 mass-% of poly(2-hydroxyethyl methacrylate) was used in an experiment with an animal infected with coccobacillus Staphylococcus aureaus. The haemoperfusion was carried out for 1 h at a rate of 20 ml/min. After 24 h of cultivation, the bacteria were isolated from a culture medium in the column.

## Example 4

A diagnosis column having a volume of 50 ml made from polypropylene (screens with a mesh size 290 $\mu$m) and packed with active charcoal (Chemviron SC XII) with untreated surface was used in an experiment with an animal infected with coccobacillus Staphylococcus aureus. The haemoperfusion was carried out for 1 h at a rate of 20 ml/min. The bacteria were then isolated from a culture medium in the column after 24 h of cultivation.

## Example 5

A diagnosis column having a volume of 150 ml made from poly(tetraethylene) (mesh size of 200 $\mu$m at the inlet screen and of 75 $\mu$m at the outlet screen) packed with a styrene - divinylbenzene copolymer (Synachrom E5) was used in an experiment with an animal infected with rod-like Escherichia coli bacteria. The haemoperfusion was carried out for 1,5 h at a rate of 20 ml/min. The bacteria were then isolated from a culture medium in the column after 24 h of cultivation.

## Example 6

A diagnosis column having a volume of 100 ml made from poly(tetrafluoroethylene) (mesh size of 290 $\mu$m at the inlet screen and of 150 $\mu$m at the outlet screen) and packed with a styrene - divinylbenzene copolymer (Persorb) was used in an experiment with an animal infected with rod-like Escherichia coil bacteria. The haemoperfusion was carried out for 1 h at a rate of 25 ml/min. The bacteria were then isolated from a culture medium in the column after 24 h of cultivation.

## Example 7

A diagnosis column having a volume of 300 ml made from polypropylene (screens with a mesh size of 214 $\mu$m) and packed with a styrene - divinylbenzene copolymer (Persorb) having their surface modified with 0,1 mass-% of poly(2-hydroxypropyl acrylate) was used in an experiment with an animal infected with coccobacillus Staphylococcus aureaus. The haemoperfusion was carried out for 2 h at a rate of 18 ml/min. The bacteria were then isolated from a culture medium in the column after 24 h of cultivation.

## Claims

1. A method for the determination of the presence of microorganisms in body liquors consisting in first allowing the body liquor to flow through a column packed with a sorbent and then in activation of the trapped microorganisms in a culture medium, wherein the body liquor is displaced from the column by means of physiological saline, the column is charged with a culture medium, and, after cultivation is completed, the culture medium is sucked off from the column, and the presence of microorganisms in the medium is determined by known methods.

2. Diagnosis column for performing the method according to claim 1, which is made from a biocompatible material, has a smooth inner surface, advantageously provided with longitudinal ribs, and is packed with a sorbent placed between partitions (2, 3) made of a porous material, wherein the column comprises a cylindrical body (1) and conical terminals (5), their volume ratio is within the range of 1:0,3 to 1:5, and wherein the supporting partitions (2, 3) have different porosity.

3. The diagnosis column according to claim 2, wherein the total inner volume of the column is 30 to 300 ml.

4. The diagnosis column according to claim 2 or 3, wherein the supporting partition (2) at the inlet terminal has a lower porosity and is advantageously formed from several layers having different porosity.

5. The diagnosis column according to claim 4, wherein the porosities of the individual layers of the partition (2) are 2000 to 80 μm, 150 to 50 μm, and 80 to 20 μm.

6. The diagnosis column according to one of claims 2 to 5, wherein the column is packed with a sorbent (4) the particles thereof being advantageously coated with a protective layer of a biocompatible material.

7. The diagnosis column according to claim 6, wherein a biocompatible material of acrylate or methacrylate type is used in an amount of 0,01 to 20 mass-%, based on the sorbent.

Fig. 1

Fig. 2

Fig. 3